# EUROPEAN PATENT APPLICATION

(11) **EP 3 721 924 A1**
(43) Date of publication of application: **14.10.2020**
(21) Application number: 19214179.4
(22) Date of filing: 06.12.2019
(51) Int. Cl.: A61M 5/32

(54) **ANTI-CONTAMINATION NEEDLE REMOVING DEVICE**

(30) Priority: 08.04.2019 TW 108112209
(71) Applicant: CoreBio Technologies Co., Ltd., Taoyuan City 325 (TW)
(72) Inventor: Shih, Kuo-Tsang, 325 Taoyuan City (TW)
(74) Representative: Straus, Alexander

(57) **Abstract**

A needle removing device includes a seat, a driving assembly, a removal assembly and a lift assembly. The driving assembly is disposed on the seat. The removal assembly has one end thereof connected to one end of the driving assembly away from the seat, and includes a needle winding member and a pressing member. The needle winding member is located on one end of the removal assembly away from the driving assembly. The pressing member is connected to the needle winding member. The lift assembly is disposed on the seat. The driving assembly and the removal assembly are movable relative to the seat via the lift assembly. The needle winding member includes a first rod and a second rod, wherein the first rod and the second rod are adjacently spaced to form a disassembly space. The pressing member includes a first elastic element and a second elastic element. The first elastic element is connected to the first rod, the second elastic element is connected to the second rod, and the first elastic element and the second elastic element cause the first rod and the second rod to be inclined close to each other. The first elastic element and the second elastic element cause the first rod and the second rod to clamp the needle, and the first rod and the second rod rotate to wind the needle.

## Description

### BACKGROUND OF THE INVENTION

### FIELD OF THE INVENTION

The present invention relates to needle removing devices and, more particularly, to a needle removing device thoroughly and effectively separating a needle from a syringe.

### DESCRIPTION OF THE PRIOR ART

In medical practice, needles and syringes are frequently used medical tools, and are both used for medical behaviors for whether blood drawing or medicine injection. For example, in a process of drawing blood, blood of a patient is generally obtained by piercing the vein with a needle. However, whether a patient has an infectious disease is unknown before such blood test, and thus, in order to prevent cross-infection, a needle can used only once instead of being repeatedly used. Centralized handling is often needed for these large amounts of used needles. During the process of recycling or handling, accidents of medical staff inadvertently being pricked by needles are inevitable, resulting in physical wounds in minor cases, or infection by residual virus or bacteria on the needles in more severe cases.

There is currently a needle removing device capable of removing a needle from a syringe to facilitate subsequent recycling and handling. However, due to different lengths and thicknesses of needles, such current needle removing device may not be suitable for needles of various sizes. Furthermore, while the device removes a needle, a function of totally and effectively removing an iron element and a plastic element cannot be achieved. Also, some needle removing devices adopt a cutting means, but which does not totally and effectively separate an iron element and a plastic element. There are some needle removing devices that separate needles by means of melting. However, such melting means can cause possible air dust pollution, and unavoidably leads to residual blood, body fluid or splashed glue on the needles. The splashed or stirred substances can not only result in environmental pollution but also splash on medical staff, bringing about risks of disease infection.

### SUMMARY OF THE INVENTION

In view of the above, the present invention provides a needle removing device suitable for removing needles of all sizes, and totally and effectively separating needles without causing air dust pollution, thus preventing substances from being splashed to the outside of the device during the process of needle removal.

According to an embodiment of the present invention, a needle removing device is for removing a needle and a syringe connected to each other. The needle removing device includes a seat, a driving assembly and a removal assembly. The driving assembly is disposed on the seat. The removal assembly has one end thereof connected to one end of the driving assembly away from the seat, and includes a needle winding member and a pressing member. The needle winding member is disposed on one end of the removal assembly away from the driving assembly. The pressing member is connected to the needle winding member. The needle winding member includes a first rod and a second rod, wherein the first rod and the second rod are adjacently spaced to form a disassembly space. The pressing member includes a first elastic element and a second elastic element. The first elastic element is connected to the first rod, the second elastic element is connected to the second rod, and the first elastic element and the second elastic element cause the first rod and the second rod to be inclined close to each other. When the driving assembly drives the removal assembly to rotate and a part of the needle is located in the disassembly space, the first elastic element and the second elastic element cause the first rod and the second rod to clamp the needle, and the first rod and the second rod rotate to wind the needle, such that the needle and the syringe are separated from each other.

According to an embodiment of the present invention, the pressing member further includes a first pressing element and a second pressing element. The first pressing element is provided on the first rod, the second pressing element is provided on the second rod, the first elastic element elastically abuts against the first pressing element, the second elastic element elastically abuts against the second pressing element.

According to an embodiment of the present invention, the first pressing element and the second pressing element are semi-circular protrusions protrudingly provided on the first rod and the second rod, respectively.

According to an embodiment of the present invention, the removal assembly further includes a sleeve, the first rod and the second rod are sleeved in the sleeve, and the sleeve limits the space between the first rod and the second rod.

According to an embodiment of the present invention, the needle removing device further includes a lift assembly. The lift assembly is disposed on the seat, and the driving assembly and the removal assembly are movable relative to the seat via the lift assembly.

According to an embodiment of the present invention, the lift assembly includes a lift platform. The lift platform is movably connected to the seat, and the driving assembly and the removal assembly are connected to the lift platform.

According to an embodiment of the present invention, the lift assembly further includes a lift shaft and an elastic restoring element. The lift shaft and the elastic restoring element are disposed between the lift platform and the seat, the lift platform is movable relative to the seat along the lift shaft, and the elastic restoring element causes the lift platform to incline away from the seat.

According to an embodiment of the present invention, the needle removing device further includes a valve assembly. The valve assembly includes a pair of valve door panels and a pair of valve springs. The pair of valve springs are respectively connected to the pair of valve door panels, and the pair of valve door panels are provided corresponding in position to the disassembly space and can open and close via the pair of valve springs.

According to an embodiment of the present invention, the needle removing device further includes a fixing support, and the fixing support is disposed on the seat. The valve assembly further includes a pair of fixing bases, the pair of fixing bases are located on the fixing support, and the pair of valve springs are respectively connected to the pair of fixing bases.

According to an embodiment of the present invention, the fixing support includes a limiting groove, and the first rod and the second rod are inserted in the limiting groove.

According to an embodiment of the present invention, the needle removing device further includes a housing. The housing and the seat are assembled to each other, the housing includes a through hole, and the through hole is disposed corresponding in position to the pair of valve door panels.

According to an embodiment of the present invention, the needle removing device further includes a needle withdrawing plate. The needle withdrawing plate is located between the first rod and the second rod, and is for entering the disassembly space to push the needle located in the disassembly space so as to separate the needle from the disassembly space.

In conclusion, in the needle removing device according to the embodiments of the present invention, the removal assembly can rotate to wind the needle so as to separate the needle and a syringe from each other, and the removal assembly can ascend and descend via the lift assembly to adapt to needles of different lengths. In addition, the pressing member can have the first rod and the second rod clamp the needle so as to adapt to needles of different thicknesses. The valve assembly can block splashed substances. Accordingly, the needle removing device of the present invention is suitable for removing needles of various sizes, and provides better applicability and convenience. Moreover, the needle removing device of the present invention can prevent substances from splashing to the outside of the device during the process of needle removal, thereby reducing environmental pollution as well as preventing medical staff from being infected.

Detailed features and advantages of the present invention are described in detail by way of embodiments below. The disclosure of the embodiments is sufficient for a person skilled in the art to understand the technical content of the present invention and accordingly implement the present invention. In addition, on the basis of the disclosed content, claims and drawings of this specification, a person skilled in the art could easily appreciate the objects and advantages of the present invention.

### BRIEF DESCRIPTION OF THE DRAWINGS

FIG. 1 is a perspective diagram of a needle removing device according to an embodiment of the present invention;
FIG. 2 is an exploded schematic diagram of partial components of a needle removing device according to an embodiment of the present invention;
FIG. 3 is a cross-sectional diagram taken along section line 3-3 of FIG. 2;
FIG. 4 is a partial enlarged and exploded diagram of a needle removing device according to an embodiment of the present invention;
FIG. 5 is a schematic diagram of a removal assembly removing a needle according to an embodiment of the present invention;
FIG. 6 is a partial enlarged diagram of a needle removing device according to an embodiment of the present invention; and
FIG. 7 is a schematic diagram a needle removing device according to an embodiment of the present invention from another viewing angle.

### DETAILED DESCRIPTION OF THE EMBODIMENTS

It is to be noted that, unless otherwise specified, the technical and scientific terms used herein refer to common meanings generally understood by a person skilled in the art. Furthermore, the term "a" used in the description refers to a quantity of at least one (one or more) unless otherwise specified. It should also be noted that, the descriptive terms related to orientation (e.g., "front", "back", "upper", and "lower", etc.) used in the description are illustrative or relative terms which need to be conceptively adjusted according to different users or different usages, and are not enumerative or absolute terms.

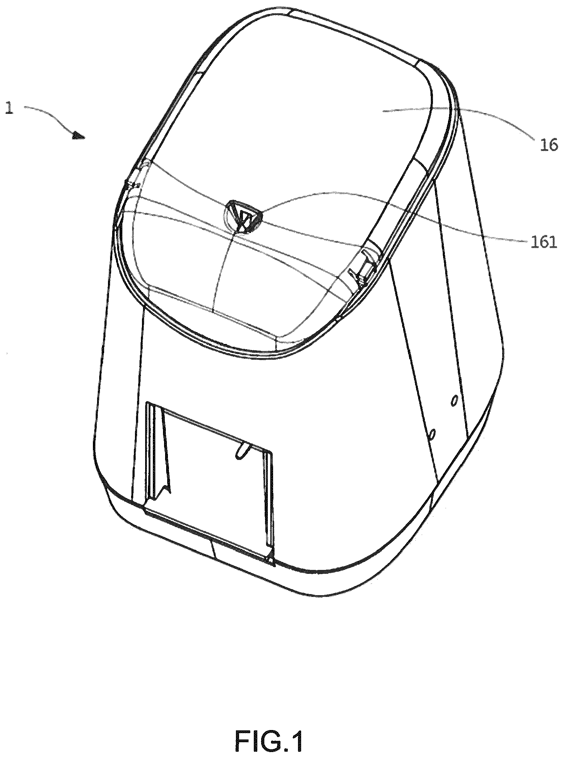

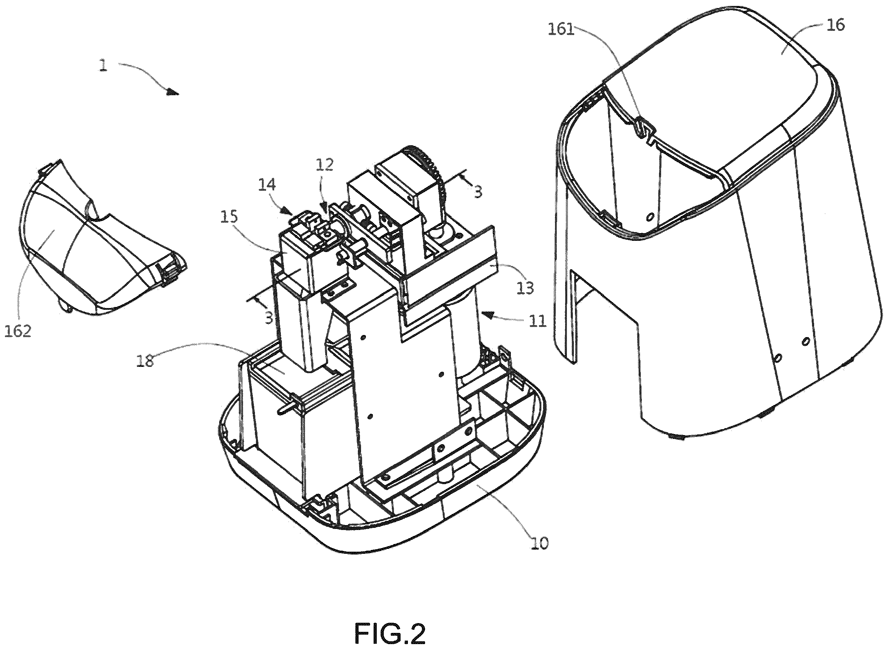

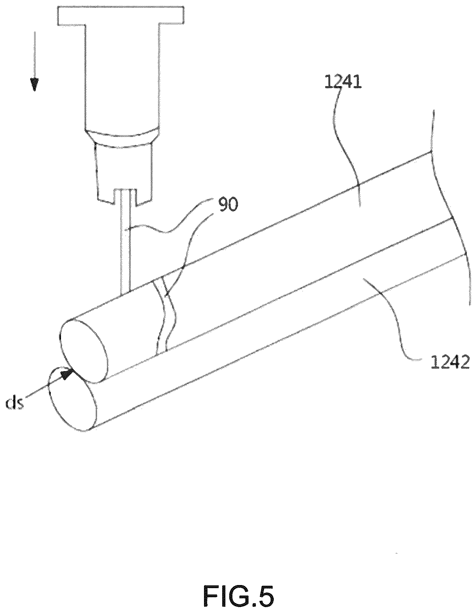

Referring to FIG. 1 and FIG. 2, FIG. 1 shows a perspective schematic diagram of a needle removing device 1 according to an embodiment of the present invention, and FIG. 2 shows an exploded schematic diagram of partial components of the needle removing device 1 according to an embodiment of the present invention. In this embodiment, the needle removing device 1 is for removing a needle 90 and a syringe 91 connected to each other (for example, as shown in FIG. 5). The needle removing device 1 includes a seat 10, a driving assembly 11, a removal assembly 12, a lift assembly 13, a valve assembly 14, a fixing support 15 and a housing 16. The fixing support 15 is fixed on the seat 10, and the housing 16 and the seat 10 are assembled to each other. The driving assembly 11, the removal assembly 12, the lift assembly 13, the valve assembly 14 and the fixing support 15 are disposed in an accommodating space formed by the housing 16 and the seat 10. The housing 16 includes a through hole 161 and an upper cover 162. The through hole 161 is provided corresponding in position to the removal assembly 12 and the valve assembly 14, and the upper cover 162 can be separated from the housing 16, so as to facilitate a user to clean components located inside the housing 16.

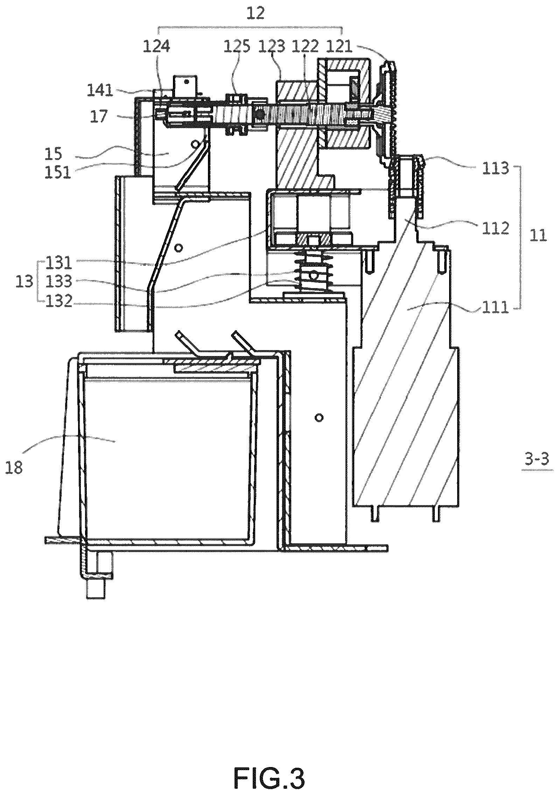

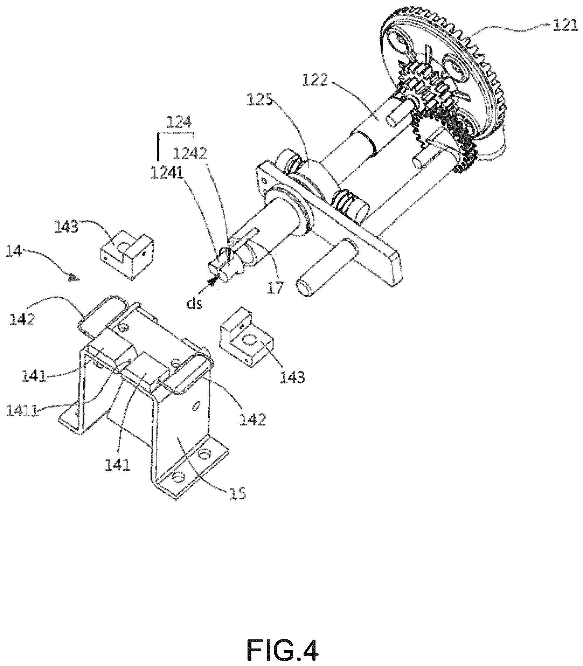

Again referring to FIG. 3 and FIG. 4, FIG. 3 shows a cross-sectional diagram taken along section line 3-3 of FIG. 2, and FIG. 4 shows a partial enlarged and exploded diagram of the needle removing device 1 according to an embodiment of the present invention. In the drawings, some components are not depicted for better illustrations. In this embodiment, the driving assembly 11 is disposed on the seat 10, and one end of the removal assembly 12 is connected to one end of the driving assembly 11 away from the seat 10. The driving assembly 11 includes a motor 111, a first transmission rod 112 and a first gear 113. One end of the first transmission rod 112 is connected to the motor 111. The first gear 113 is provided on one end of the first transmission rod 112 away from the motor 111. The removal assembly 12 includes a second gear 121, a second transmission rod 122, a linking member 123, a needle winding member 124 and a pressing member 125. The second gear 121 is engaged with the first gear 113 of the driving assembly 11, and is provided on one end of the second transmission rod 122. The second transmission rod 122 passes through the linking member 123. The needle winding member 124 is located on one end of the removal assembly 12 away from the driving assembly 11, and the pressing member 125 is connected to the needle winding member 124.

As shown in FIG. 4, in this embodiment, the needle winding member 124 includes a first rod 1241 and a second rod 1242. The first rod 1241 and the second rod 1242 are located on one end of the second transmission rod 122 away from the second gear 121 and adjacently arranged next to each other, and extend towards the same axial direction from the second transmission rod 122. Furthermore, the first rod 1241 and the second rod 1242 are spaced from each other to form a disassembly space ds; that is, the disassembly space ds is located between the first rod 1241 and the second rod 1242. As shown in FIG. 2 to FIG. 4, the through hole 161 is aligned with the first rod 1241, the second rod 1242 and the disassembly space ds.

Referring to FIG. 5, FIG. 5 shows a schematic diagram of the removal assembly 12 removing the needle 90 according to an embodiment of the present invention. In the drawing, some components are not depicted for better illustrations. As shown in FIG. 3 to FIG. 5, if a user needs to remove the needle 90 from the syringe 91, he/she can insert the needle 90 into the through hole 161 (refer to the direction of the arrow in FIG. 5), and have a part of the needle 90 enter the disassembly space ds between the first rod 1241 and the second rod 1242. In the operation of the needle removing device 1, the driving assembly 11 drives the removal assembly 12 to rotate; that is, the motor 111 drives the first transmission rod 112 and the first gear 113 to rotate, the first gear 113 drives the second gear 121 and the second transmission rod 122 to rotate, and the second transmission rod 122 drives the first rod 1241 and the second rod 1242 to rotate around the axis of the second transmission rod 122. At this point in time, the first rod 1241 and the second rod 1242 wind the needle 90, such that the needle 90 is wound on the first rod 1241 and the second rod 1242 and separates from the syringe 91.

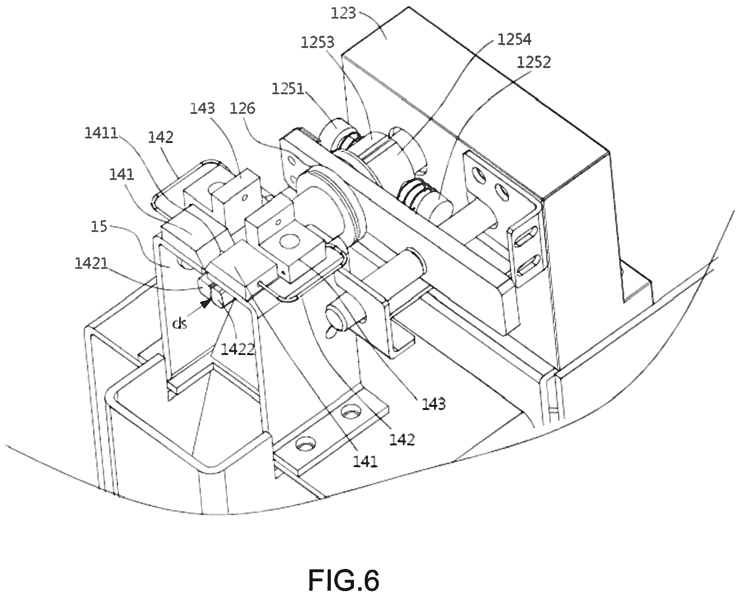

Referring to FIG. 6, FIG. 6 shows a partial enlarged diagram of a needle removing device according to an embodiment of the present invention. In the drawings, some components are not depicted for better illustrations. As shown in FIG. 3, FIG. 4 and FIG. 6, in this embodiment, the pressing member 125 is located between the needle winding member 124 and the linking member 123, and includes a first elastic element 1251 and a second elastic element 1252. The first elastic element 1251 is connected to the first rod 1241, the second elastic element 1252 is connected to the second rod 1242, and elastic restoring forces of the first elastic element 1251 and the second elastic element 1252 are continuously applied on the first rod 1241 and the second rod 1242, such that the first rod 1241 and the second rod 1242 are inclined close to each other. Due to different thicknesses of the needle 90 to be removed, when a thicker needle 90 enters the disassembly space ds between the first rod 1241 and the second rod 1242 (for example, when the diameter of the needle 90 is greater than the width of the disassembly space ds in a normal state of not receiving any force), the needle 90 stretches the first rod 1241 and the second rod 1242, and the first rod 1241 and the second rod 1242 then tightly clamp the needle 90 by the restoring forces generated from elastic deformation thereof. If a thinner needle 90 enters the disassembly space ds between the first rod 1241 and the second rod 1242 (for example, when the diameter of the needle 90 is smaller than the width of the disassembly space ds in a normal state of not receiving any force), the forces applied by the first elastic element 1251 and the second elastic element 1252 can still have the first rod 1241 and the second rod 1242 clamp the thinner needle 90 tightly. Given that the needle 90 is securely clamped by the first rod 1241 and the second rod 1242, the first rod 1241 and the second rod 1242 are capable of successfully winding the needle 90.

As shown in FIG. 6, in this embodiment, the pressing member 125 further includes a first pressing element 1253 and a second pressing element 1254. The first pressing element 1253 is provided on the first rod 1241, the second pressing element 1254 is provided on the second rod 1242, the first elastic element 1251 elastically abuts against the first pressing element 1253, and the second elastic element 1252 elastically abuts against the second pressing element 1254. The first pressing element 1253 and the second pressing element 1254 receive forces of the first elastic element 1251 and the second elastic element 1252 and can thus drive the first rod 1241 and the second rod 1242 to move close to each other. In this embodiment, the first pressing element 1253 is a semi-circular protrusion protrudingly provided on the first rod 1241, the second pressing element 1254 is a semi-circular protrusion protrudingly provided on the second rod 1242, and the first pressing element 1253 and the second pressing element 1254 jointly form a circular contour. Via the first pressing element 1253 and the second pressing element 1254, the stresses applied by the first elastic element 1251 and the second elastic element 1252 on the first rod 1241 and the second rod 1242 can be uniformly distributed.

As shown in FIG. 6, in this embodiment, the removal assembly 12 further includes a sleeve 126, the first rod 1241 and the second rod 1242 are sleeved in the sleeve 126, and the sleeve 126 limits the space between the first rod 1241 and the second rod 1242. In other words, the sleeve 126 limits the maximum aperture of the disassembly space ds when the first rod 1241 and the second rod 1242 are stretched open, hence preventing an overly thick object from being mistakenly inserted between the first rod 1241 and the second rod 1242 and causing damage.

As shown in FIG. 4 and FIG. 6, in this embodiment, the valve assembly 14 includes a pair of valve door panels 141 and a pair of valve springs 142. The pair of valve springs 142 are respectively connected to the pair of valve door panels 141. The pair of valve door panels 141 are provided corresponding in position to the disassembly space ds, and can be elastically opened and closed via the pair of valve springs 142. The valve door panels 141 are provided corresponding in position to the opening of the through hole 161 near the first rod 1241 and the second rod 1242, that is, the valve door panels 141 can block between the through hole 161 and the first rod 1241 and the second rod 1242. When the needle 90 is inserted into the through hole 161, the needle 90 stretches open the pair of valve door panels 141 and enters between the first rod 1241 and the second rod 1242, and the pair of valve door panels 141 receives the elastic restoring forces of the valve springs 142 and maintain a minimum aperture. Thus, when the first rod 1241 and the second rod 1242 wind the needle 90, the residual substances on the needle 90 can be blocked by the valve door panels 141 and are not easily splashed out.

As shown in FIG. 4 and FIG. 6, in this embodiment, the pair of valve door panels 141 include door panel holes 1411 which are disposed on the connecting sides of the pair of valve door panels 141 and are aligned with the disassembly space ds. When the diameter of the needle 90 is smaller than the aperture of the door panel holes 1411, the needle 90 can pass through the pair of valve door panels 141 from the door panel holes 1411 without stretching the pair of valve door panels 141. When the diameter of the needle 90 is slightly larger than the aperture of the door panel holes 1411, the needle 90 can pass through the pair of valve door panels 141 from the door panel holes 1411 by slightly stretching the pair of valve door panels 141. Thus, when the needle 90 is removed, the valve door panels 141 can be kept at an aperture as small as possible, so as to reduce the possibility of splashing the residual substances on the needle 90.

As shown in FIG. 4 and FIG. 6, in this embodiment, the valve assembly 14 further includes a pair of fixing bases 143. The pair of fixing bases 143 are fixed on the fixing support 15, and the pair of valve springs 142 are respectively connected to the pair of fixing bases 143, that is, two ends of each valve spring 142 are respectively connected to the corresponding fixing base 143 and valve door panel 141, and the pair of valve door panels are movably disposed on the fixing support 15.

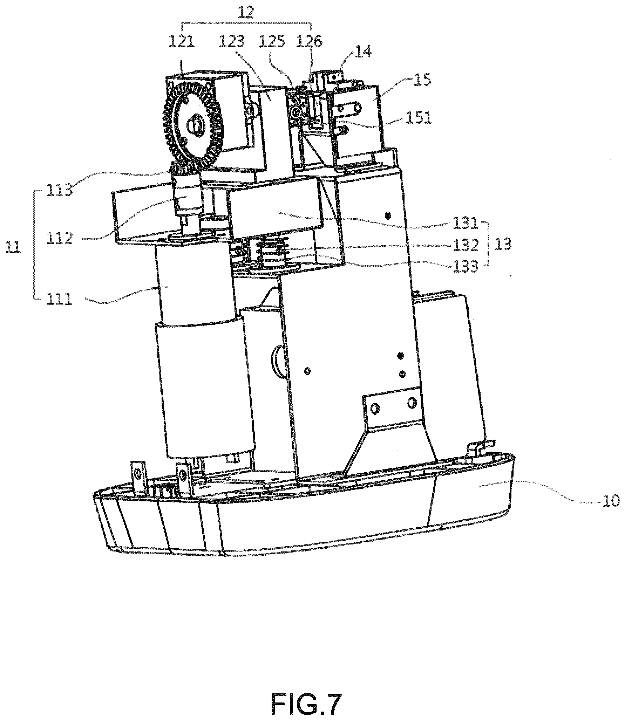

Referring to FIG. 7, FIG. 7 shows a schematic diagram of the needle removing device 1 according to an embodiment of the present invention from another viewing angle. In the drawings, some components are not depicted for better illustrations. As shown in FIG. 2, FIG. 3 and FIG. 7, in this embodiment, the lift assembly 13 is disposed on the seat 10, and includes a lift platform 131. The lift platform 131 is movably connected to the seat 10, and the driving assembly 11 and the removal assembly 12 are connected to the lift platform 131. The driving assembly 11 and the removal assembly 12 are movable relative to the seat 10 via the lift assembly 13; that is, the driving assembly 11 and the removal assembly 12 are capable of ascending and descending. In contribution to the lift design of the driving assembly 11 and the removal assembly 12, the first rod 1241 and the second rod 1242 are also capable of ascending and descending correspondingly. Thus, when the removal assembly 12 removes the needle 90 of different lengths, the first rod 1241 and the second rod 1242 can also correspondingly ascend and descend in accordance with the length of the needle 90, so as to completely wind the needle 90.

As shown in FIG. 3 and FIG. 7, in this embodiment, the lift assembly 13 further includes a lift shaft 132 and an elastic restoring element 133. The lift shaft 132 and the elastic restoring element 133 are located between the lift platform 131 and the seat 10, the lift platform 131 is movable relative to the seat 10 along the lift shaft 132, and the elastic restoring element 133 causes the lift platform 131 to incline away from the seat 10. The elastic restoring element 133 is, for example but not limited to, a spring winding on the lift shaft 132. When the needle 90 inserts between the first rod 1241 and the second rod 1242 and the first rod 1241 and the second rod 1242 wind the needle 90, a user can continuously apply a force on the syringe 91 so as to have the needle 90 move towards the direction of the seat 10. At this point in time, the driving assembly 11 and the removal assembly 12 are pressed and are prone to descending; however, the elastic restoring force of the elastic restoring element 133 causes the driving assembly 11 and the removal assembly 12 to maintain the height or to even ascend, so as to facilitate the first rod 1241 and the second rod 1242 to continue winding the needle 90 such that the needle 90 can be completely wound. Once the needle 90 is removed, the elastic restoring element 133 can cause the driving assembly 11 and the removal assembly 12 to return to an initial position.

As shown in FIG. 3 and FIG. 7, in this embodiment, the fixing support 15 includes a limiting groove 151, and the first rod 1241 and the second rod 1242 are inserted in the limiting groove 151. For example, the sleeve 126 sleeving outside the first rod 1241 and the second rod 1242 is inserted in the limiting groove 151, and the limiting groove 151 matches the sleeve 126 with respect to the width direction, and has a space for the sleeve 126 to move in the height direction. With the matching between the sleeve 126 and the limiting groove 151, the ascending and descending range of the removal assembly 12 relative to the seat 10 is limited.

As shown in FIG. 3 and FIG. 4, in this embodiment, the needle removing device 1 further includes a needle withdrawing plate 17. The needle withdrawing plate 17 is located between the first rod 1241 and the second rod 1242. After the first rod 1241 and the second rod 1242 wind the needle 90 and the needle 90 is separated from the syringe 91, the needle withdrawing plate 17 can enter the disassembly space ds to push the needle 90 located in the disassembly space ds so as to cause the needle 90 to depart from the disassembly space ds. The needle withdrawing plate 17 can be linked by the rotational movement of the current linking mechanism and the second transmission rod 122. For example but not limited to, after the second transmission rod 122 drives the first rod 1241 and the second rod 1242 to rotate by 360 degrees, a corresponding flange on the second gear 121 or the second transmission rod 122 ejects the needle withdrawing plate 17. In different embodiments, the needle withdrawing plate 17 can also be operated manually.

As shown in FIG. 2 and FIG. 3, in this embodiment, the needle removing device 1 can further include an accommodating container 18. The accommodating container 18 is provided on the seat 10, and is aligned with the first rod 1241, the second rod 1242 and the fixing support 15, and can collect the needle 90 having been removed. For example, after the needle 90 is wound by the first rod 1241 and the second rod 1242 and is ejected out of the disassembly space ds by the needle withdrawing plate 17, the wound needle 90 is moved along the channel between the fixing support 15 and falls into the accommodating container 18.

In conclusion, the needle removing device according to the embodiments of the present invention is capable of rotating the removal assembly by the driving assembly and winding the needle by the first rod and the second rod of the removal assembly, so as to separate the needle and the syringe from each other. Furthermore, the lift assembly enables the driving assembly and the removal assembly to move up and down, so as to adapt to needles of different lengths. The pressing member can apply a force on the first rod and the second rod to have the first rod and the second rod incline close to each other, so as to adapt to needles of different thicknesses. The valve assembly can keep at an aperture as small as possible during the process of winding the needle, so as to block splashed substances. Once the needle is separated from the syringe, the valve assembly can be automatically closed. Therefore, the needle removing device is suitable for removing needles of various sizes, and provides better applicability and convenience. In addition, the needle removing device of the present invention can prevent substances from splashing to the outside of the device during the process of removing the needle, thus reducing environmental pollution and preventing medical staff from being infected.

Although the technical content of the present invention is disclosed by way of preferred embodiments as above, it is to be understood that the scope of the present invention is not limited thereto. Slight modifications and variations made without departing from the spirit of the present invention by a person skilled in the art are to be encompassed within the scope of the present invention, and therefore the legal protection of the present invention shall be defined by the appended claims.

## Claims

1. A needle removing device, for removing a needle and a syringe connected to each other, comprising:
a seat;
a driving assembly disposed on the seat;
a removal assembly, one end of the removal assembly being connected to one end of the driving assembly away from the seat, the removal assembly comprising a needle winding member and a pressing member, the needle winding member disposed on one end of the removal assembly away from the driving assembly, the pressing member being connected to the needle winding member; wherein, the needle winding member comprises a first rod and a second rod, the first rod and the second rod are adjacently spaced to form a disassembly space, the pressing member comprises a first elastic element and a second elastic element, the first elastic element is connected to the first rod, the second elastic element is connected to the second rod, and the first elastic element and the second elastic element cause the first rod and the second rod to be inclined close to each other; and
a lift assembly disposed on the seat, the driving assembly and the removal assembly being movable relative to the seat via the lift assembly;
wherein, the pressing member further comprises a first pressing element and a second pressing element, the first pressing element is provided on the first rod, the second pressing element is provided on the second rod, the first elastic element elastically abuts against the first pressing element, and the second elastic element elastically abuts against the second pressing element;
wherein, when the driving assembly drives the removal assembly to rotate and a part of the needle is located in the disassembly space, the first elastic element and the second elastic element cause the first rod and the second rod to clamp the needle, and the first rod and the second rod rotate to wind the needle, such that the needle and the syringe are separated from each other.

2. The needle removing device according to claim 1, wherein the first pressing element and the second pressing element are semi-circular protrusions protrudingly provided on the first rod and the second rod, respectively.

3. The needle removing device according to claim 1, wherein the removal assembly further comprises a sleeve, the first rod and the second rod are sleeved in the sleeve, and the sleeve limits a space between the first rod and the second rod.

4. The needle removing device according to claim 1, wherein the lift assembly comprises a lift platform, the lift platform is movably connected to the seat, and the driving assembly and the removal assembly are connected to the lift platform.

5. The needle removing device according to claim 4, wherein the lift assembly further comprises a lift shaft and an elastic restoring element, the lift shaft and the elastic restoring element are located between the lift platform and the seat, the lift platform is movable relative to the seat along the lift shaft, and the elastic restoring element causes the lift platform to be inclined away from the seat.

6. The needle removing device according to claim 1, further comprising:
a valve assembly, comprising a pair of valve door panels and a pair of valve springs, wherein the pair of valve springs respectively connected to the pair of valve door panels, the pair of valve door panels is disposed corresponding in position to the disassembly space, and the pair of valve door panels is capable of opening and closing via the pair of valve springs.

7. The needle removing device according to claim 6, further comprising:
a fixing support disposed on the seat;
wherein, the valve assembly further comprises a pair of fixing bases, the pair of fixing bases are disposed on the fixing support, and the pair of valve springs are respectively connected to the pair of fixing bases.

8. The needle removing device according to claim 7, wherein the fixing support comprises a limiting groove, and the first rod and the second rod are inserted in the limiting groove.

9. The needle removing device according to claim 6, further comprising:
a housing, the housing and the seat being assembled to each other, the housing comprising a through hole, and the through hole is disposed corresponding in position to the pair of valve door panels.

10. The needle removing device according to claim 1, further comprising:
a needle withdrawing plate located between the first rod and the second rod, for entering the disassembly space so as to push the needle located in the disassembly space to separate the needle from the disassembly space.
